# EUROPEAN PATENT SPECIFICATION

(11) **EP 3 066 990 B1**
(45) Date of publication and mention of the grant of the patent: **31.01.2018**
(21) Application number: 16159831.3
(22) Date of filing: 11.03.2016
(51) Int. Cl.: A61B 17/04, A61B 17/12, A61B 17/00, A61B 17/29

(54) **LIGATION AND SPECIMEN RETRIEVAL DEVICE**
LIGATIONS- UND PROBENGEWINNUNGSVORRICHTUNG
DISPOSITIF D'EXTRACTION D'ÉCHANTILLONS ET DE LIGATURE

(30) Priority: 12.03.2015 US 201562131970 P; 19.02.2016 US 201615047972
(43) Date of publication of application: 14.09.2016
(73) Proprietor: Covidien LP, Mansfield, MA 02048 (US)
(72) Inventor: Syed Ahmed, Mushtaque, 500 057 Hyderabad (IN); Shankarsetty, Jeevan M., 560096 Nandini Layout (IN)
(74) Representative: Soames, Candida Jane

(56) References cited:
- WO-A1-2016/135192
- US-A- 5 190 555
- US-A- 5 647 372
- US-A- 5 749 879
- US-A- 6 007 546
- US-A1- 2012 158 010

## Description

### BACKGROUND

### 1. Technical Field

The present disclosure is directed to a specimen retrieval device for surgical use. More particularly, the present disclosure is directed to a specimen retrieval device including a pouch and at least a pair of suture loops for drawing tissue into the pouch.

### 2. Background of Related Art

Endoscopic surgical devices have been developed to perform surgical procedures through small incisions in the skin or through small diameter cannulas. These surgical procedures minimize trauma to the patient as compared to conventional open surgical procedures to decrease recovery time and minimize costs associated with the surgical procedures.

Endoscopic surgical devices are designed to perform a variety of surgical procedures. Typically, these devices include distally located end effectors that are configured to perform a particular surgical operation on tissue, e.g., suturing, dissection, collection, etc..., and a proximally located actuator adapted to operate the end effector remotely. The end effector is dimensioned to be received through the small diameter opening in tissue and/or the small diameter cannula to access a surgical site.

Specimen retrieval devices for retrieving tissue removed from within a patient during a surgical procedure, e.g., an appendectomy, are well known. Typically, specimen retrieval devices include a bag or pouch that is supported on a support shaft. The bag or pouch can be deployed from within an outer tube of the device to a position adjacent the surgical site to collect tissue, that has been transected or dissected within the patient. Surgical devices are also known for transecting or dissecting tissue endoscopically during such an endoscopic surgical procedure. A surgical procedure that requires tissue to be both dissected and collected requires two incisions/ cannulas to access the surgical site with a retrieval device and a transection device or, alternatively, requires removal of the transection device from an incision/cannula prior to insertion of the retrieval device. The use of additional instruments and/or additional access cannulas or ports increases the possibility of infection and causes additional trauma to the patient. Similarly, the removal of an endoscopic device and subsequent insertion of a second endoscopic device increases the likelihood of infection. US2012/0158010 A1 discloses a specimen retrieval device including an applicator and a pouch which is operably disposed within the applicator and is deployable therefrom. The pouch defines a longitudinal axis therethrough and includes a closed bottom portion and an open upper portion. A drawstring is operably coupled to the upper portion.

WO 2016/135192 A falling under the provisions of Article 54(3) EPC discloses a device for cutting and removing tissue having a specimen pouch with a pull cord attached to the opening thereof for closing the specimen pouch, and at least one, in particular several cutting wires that are arranged inside the specimen pouch. After introducing the tissue into the specimen pouch located in the abdominal cavity of a patient, and closing the specimen pouch, the cutting wires can be pulled through the tissue in order to cut it in smaller pieces in this way. The small tissue pieces can then be removed from the abdominal cavity, together with the specimen pouch.

It would be advantageous to provide a single endoscopic surgical instrument capable of performing a plurality of surgical operations.

### SUMMARY

In an aspect of the present disclosure, a ligation and specimen retrieval device is described that includes a hollow outer shaft having a proximal end and a distal end, an inner shaft extending through the outer shaft, the inner shaft being movable in relation to the outer shaft from a retracted position to an advanced position, and a pouch defining a receptacle and having an open end and a closed end. The ligation and specimen retrieval device also includes at least one suture member extending from the proximal end of the outer shaft to the distal end of the outer shaft. The at least one suture member includes a distal end having a suture loop and a proximal end that is accessible to a clinician at the proximal end of the outer shaft. The at least one suture member is adapted to be cinched about tissue upon retraction of the at least one suture member within the outer tube. The ligation and specimen retrieval device is transitionable from a non-deployed state in which the pouch and the at least one suture member are supported within a distal end of the outer shaft to a deployed state in which the pouch and the at least one suture member are positioned externally of the outer shaft.

In embodiments, the pouch is rolled up and supported in the distal end of the outer shaft in the non-deployed state.

In certain embodiments, the pouch is rolled about the at least one suture member in the non-deployed state.

In some embodiments, the at least one suture member includes a first suture member and a second suture member, wherein the first suture member has a first suture loop and the second suture member has a second suture loop.

In embodiments, the second suture loop is secured to the pouch adjacent the open end of the pouch.

In certain embodiments, the pouch includes a plurality of clips which releasably secure the second suture loop to the pouch.

In some embodiments, the clips are configured to automatically release the second suture loop upon retraction of the second suture member within the outer shaft.

In embodiments, the proximal end of the at least one suture member is secured to a knot puller and the knot puller is movably supported on the proximal end of the ligation and specimen retrieval device from an advanced position to a retracted position to retract the at least one suture member within the outer tube.

In certain embodiments, the proximal end of the first and second suture members are secured to first and second knot pullers and the first and second knot pullers are movably supported on the proximal end of the ligation and specimen retrieval device from an advanced position to a retracted position to retract the first and second suture members within the outer tube.

In some embodiments, the inner shaft defines at least one channel and the at least one suture member extends through the at least one channel from the distal end of the inner shaft to the proximal end of the inner shaft.

In certain embodiments, the ligation and specimen retrieval device includes a gripping member secured to a proximal end of the inner shaft.

In embodiments, the device includes a housing secured to a proximal end of the outer shaft. The housing has a pair of finger loops and the gripping member has a thumb loop that is configured to facilitate movement of the inner shaft in relation to the outer shaft.

In some embodiments, the device includes a pouch closure member extending through the outer shaft. The pouch closure member has a proximal end extending from a proximal end of the outer shaft and a distal end positioned about the open end of the pouch, wherein the pouch closure member is retractable within the outer shaft to cinch the open end of the pouch.

In embodiments, the proximal end of the pouch closure member includes a closure ring that is configured to be grasped by a clinician.

In another aspect of the present disclosure, a method of ligating and retrieving tissue is described that includes inserting a distal end of a ligation and specimen retrieval device through an incision in a patient to a position adjacent tissue; actuating the ligation and specimen retrieval device to deploy a pouch and at least one suture member from an outer tube of the ligation and specimen retrieval device; positioning tissue through a loop formed on the distal end of the at least one suture member; retracting the at least one suture member through the outer tube of the ligation and specimen retrieval device to cinch the loop about a portion of the tissue; positioning the tissue to be transected into a receptacle of the pouch; transecting the tissue below the loop such that a portion of the tissue is received within the receptacle of the pouch; and retracting a closure member having a distal end positioned about an open end of the pouch and a proximal end extending from the proximal end of the outer shaft to close the open end of the pouch.

In embodiments, the at least one suture member includes a first suture member and a second suture member and the step of positioning tissue through a loop formed on the distal end of the at least one suture member includes positioning tissue through a first loop formed on the distal end of the first suture member and positioning tissue through a second loop on the distal end of the second suture member.

In some embodiments, the step of retracting the at least one suture member includes retracting the first suture member to cinch the first loop about a first portion of the tissue and retracting the second suture to cinch the second loop about a second portion of the tissue.

In certain embodiments, the step of transecting the tissue includes transecting the tissue between the first loop and the second loop.

The invention is defined in claim 1.

### BRIEF DESCRIPTION OF THE DRAWINGS

Various aspects of the present disclosure are described hereinbelow with reference to the drawings, which are incorporated in and constitute a part of this specification, wherein:
Fig. 1 is a top, perspective view of the presently disclosed ligation and specimen retrieval device in a non-deployed state;
Fig. 2 is a side, exploded, perspective view of the ligation and specimen retrieval device shown in Fig. 1;
Fig. 3 is an enlarged, side, perspective view of the ligation and specimen retrieval device shown in Fig. 1 with the distal end of the outer tube shown in phantom;
Fig. 4 is a perspective view from the distal end of the distal end of the ligation and specimen retrieval device shown in Fig. 1 with a suture loop shown in phantom;
Fig. 5 is a top view of the proximal end of the ligation and specimen retrieval device shown in Fig. 1 as the inner shaft gripping member is moved towards its advanced position to move the device to the deployed state;
Fig. 6 is a perspective view of the distal end of the ligation and specimen retrieval device shown in Fig. 1 in a deployed state with a rolled pouch rolled positioned adjacent the distal end of the inner shaft and a distal end of the a suture loop shown in phantom;
Fig. 7 is a perspective view of the distal end of the ligation and specimen retrieval device shown in Fig. 1 with the pouch and suture loops deployed from the outer shaft and the pouch unrolled;
Fig. 8 is a perspective view of the proximal end of the ligation and specimen retrieval device shown in Fig. 1 in the deployed state with the inner shaft and the suture loop actuators in their advanced positions;
Fig. 9 is a perspective view of the proximal end of the ligation and specimen retrieval device shown in Fig. 1 with the inner shaft and second suture loop actuator in advanced positions and the first suture loop actuator in its retracted position to tighten the first suture loop about tissue;
Fig. 10 is a side perspective view of the distal end of the ligation and specimen retrieval device shown in Fig. 1 with the pouch and the first and second suture loops deployed, the pouch unrolled, and the first suture loop tightened about tissue;
Fig. 10A is an enlarged view of the indicated area of detail shown in Fig. 10;
Fig. 11 is a side perspective view of the distal end of the ligation and specimen retrieval device shown in Fig. 1 with the pouch and the first and second suture loops deployed, the pouch unrolled, and the first and second suture loops tightened about tissue;
Fig. 11A is an enlarged view of the indicated area of detail shown in Fig. 11;
Fig. 12 is a perspective view of the proximal end of the ligation and specimen retrieval device shown in Fig. 1 with the inner shaft in an advanced position and the first and second suture loop actuators in their retracted positions to tighten the first suture and second suture loops about tissue;
Fig. 13 is a side perspective view of the distal end of the ligation and specimen retrieval device shown in Fig. 1 with the tissue positioned within the pouch and the closure loop cinched about the open end of the pouch; and
Fig. 14 is a side perspective view of the pouch shown in Fig. 13 after the inner and outer shafts of the ligation and specimen retrieval device have been separated from the pouch and suture loops.

### DETAILED DESCRIPTION OF EMBODIMENTS

Embodiments of the present disclosure are now described in detail with reference to the drawings in which like reference numerals designate identical or corresponding elements in each of the several views. As used herein, the term "clinician" refers to a doctor, nurse, or any other care provider and may include support personnel. Throughout this description, the term "proximal" refers to the portion of the device or component of the device that is closest to the clinician and the term "distal" refers to the portion of the device or component of the device that is farthest from the clinician. In addition, as used herein the term endoscopic procedure refers to any surgical procedure conducted through a small diameter incision and/or cannula including endoscopic, laparoscopic, and arthroscopic surgical procedures. Additionally, the term endoscopic instrument includes any instrument configured to perform a surgical procedure through a small diameter incision and/or cannula including endoscopic, laparoscopic, and arthroscopic surgical procedures.

The present disclosure is directed to a ligation and specimen retrieval device that includes structure for both ligating tissue and retrieving or collecting the ligated tissue. The device enables a clinician to perform a plurality of surgical procedures with a single device to facilitate the performance of a surgical procedure more quickly to minimize trauma to the patient and the likelihood of infection.

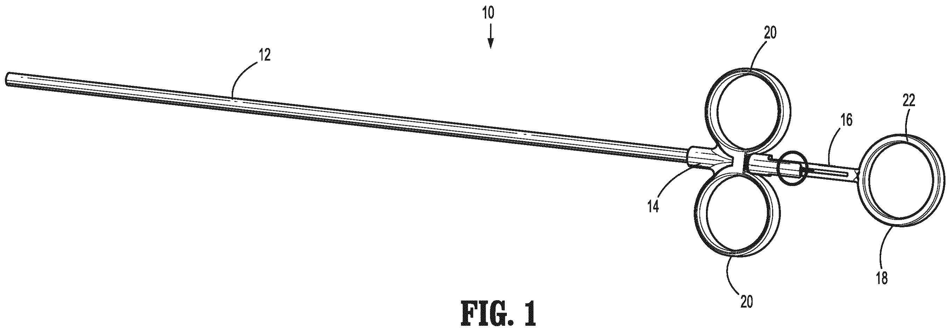

Referring to Fig. 1, the presently disclosed ligation and specimen retrieval device is indicated generally by the reference number 10 and includes a hollow outer shaft 12, a tubular housing 14 secured to a proximal end of the outer shaft 12, a hollow inner shaft 16 slidably disposed within the outer shaft 12, and a gripping member 18 secured to the proximal end of the inner shaft 16. The outer shaft 12 and housing 14, and the inner shaft 16 and the gripping member 18, respectively, can be integrally formed or secured together using known fastening techniques including welding or adhesives. In one embodiment, the housing 14 includes a pair of radially extending finger loops 20 and the gripping member 18 includes a thumb loop 22. The finger loops 20 and thumb loop 22 can be grasped by a clinician to move the inner shaft 16 in relation to the outer shaft 12 to transition the device 10 from a non-deployed state to a deployed state as will be discussed in further detail below. It is envisioned that the device 10 can include other hand grip configurations on the housing 14 and/or the inner shaft 16.

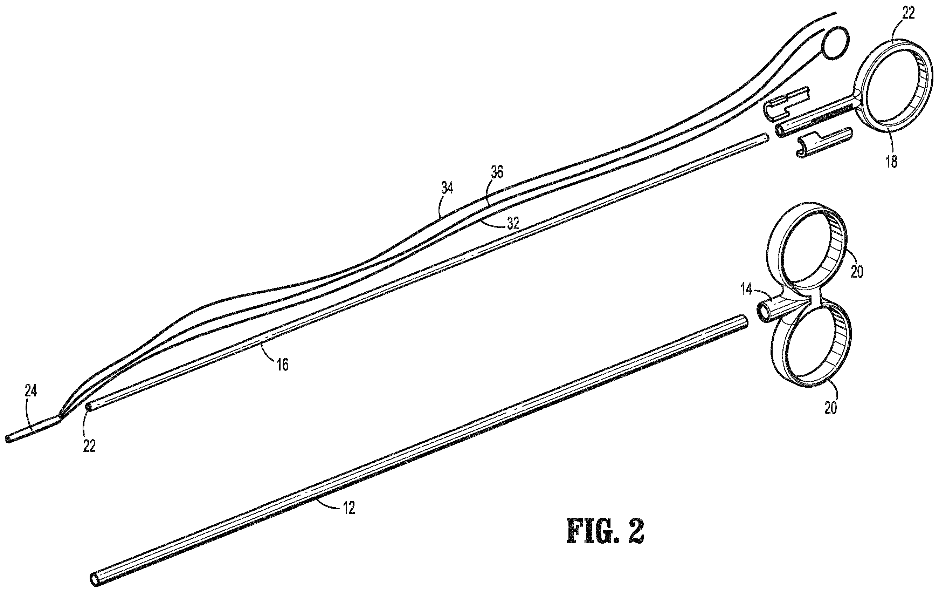

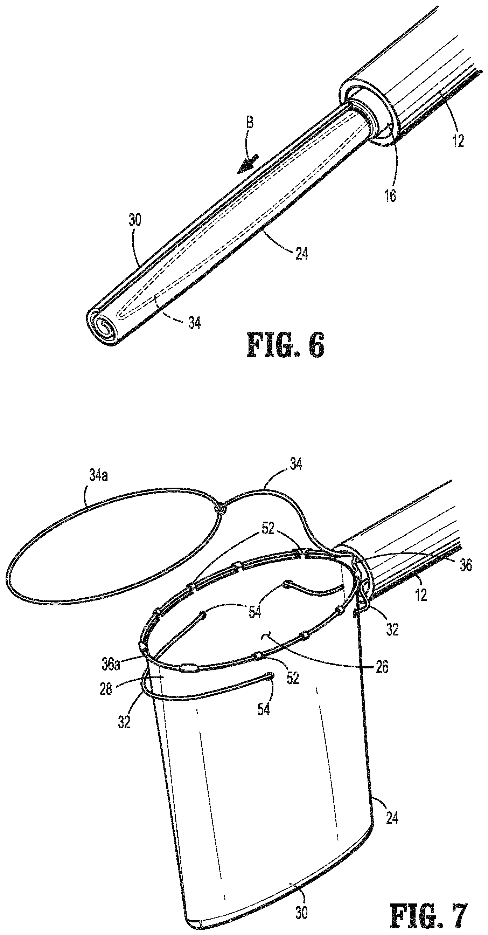

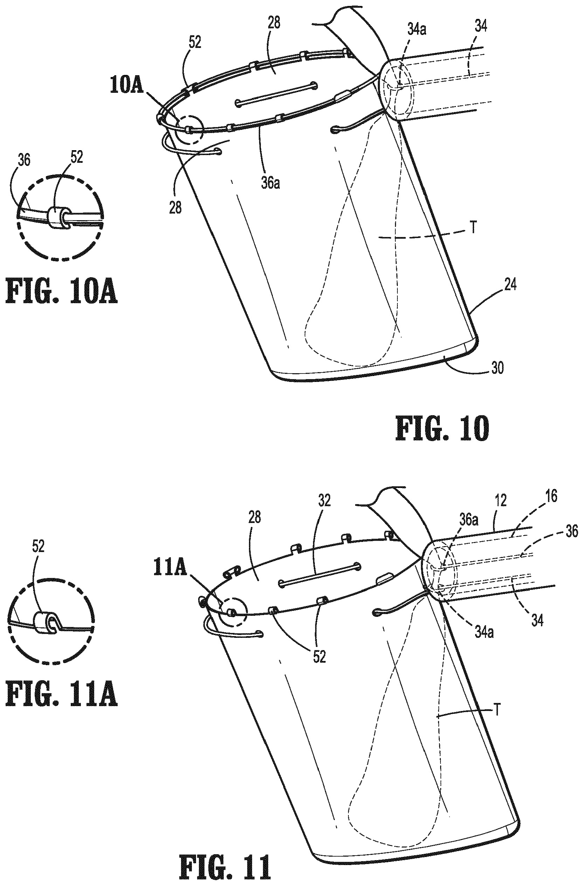

Referring to Fig. 2, the inner shaft 16 defines a plurality of internal channels 22 (not shown separately) and has a distal end positioned proximally of the distal end of the outer shaft 12. In the non-deployed state of the retrieval device 10, a pouch 24 is rolled up and supported within the distal end of the outer shaft 12. The pouch 24 defines a receptacle 26 (Fig. 7) and has an open end 28 and a closed end 30. The open end 28 of the pouch 24 supports a distal end of a pouch closure member 32 and a second suture member 36, respectively. A first suture member 34 is positioned adjacent the open end 28 of the pouch 24. Each of the closure members 32 and suture members 34 and 36 extends through a respective channel 22 of the inner shaft 16 as will be discussed in further detail below. The closure member 32 is configured to close the open end 28 of the pouch 24 and the first and second suture members 34 and 36 are configured to ligate a tissue specimen "T" (Fig. 10) as discussed in further detail below.

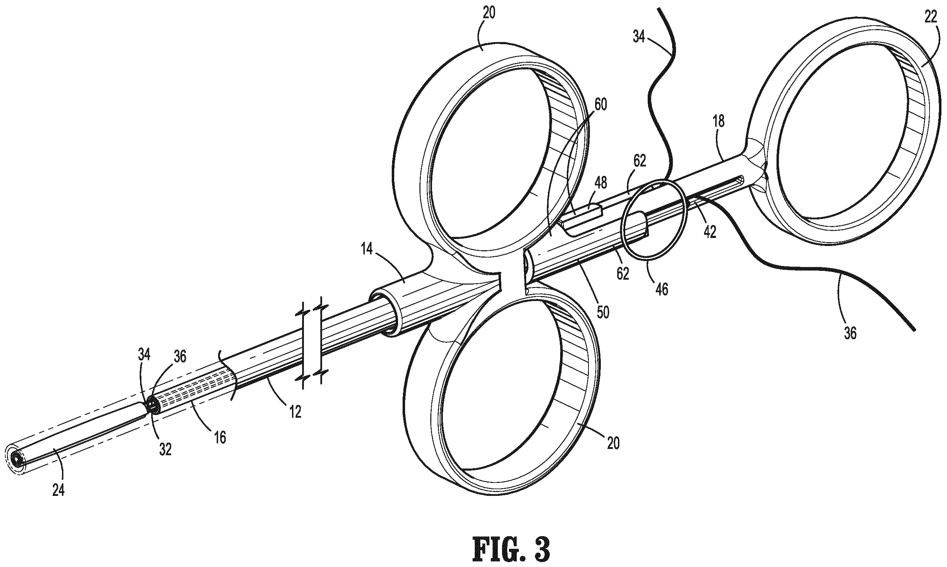

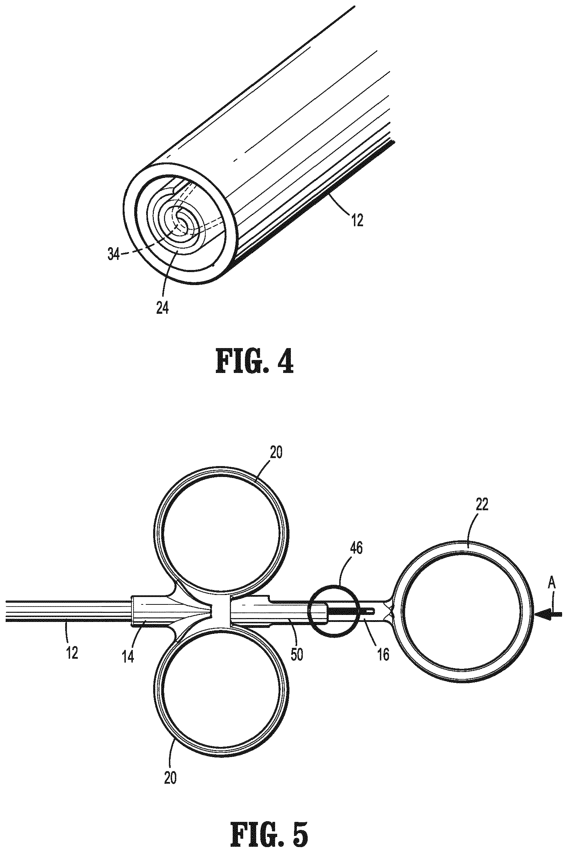

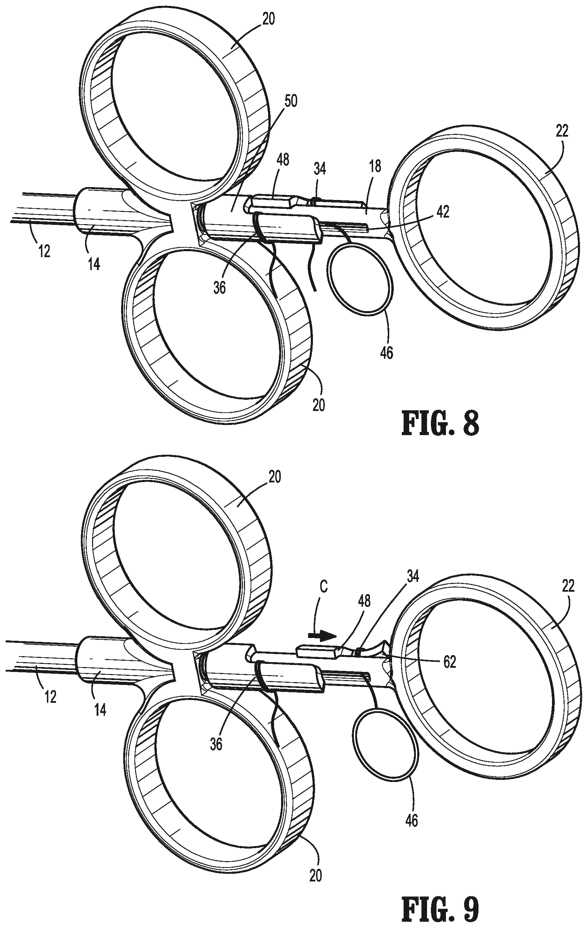

Referring also to Figs. 3-6, the inner shaft 16 is slidable within the outer shaft 12 to transition the device 10 from a non-deployed state (Fig. 3) to a deployed state (Fig. 6). In the non-deployed state shown in Figs. 3 and 4, the pouch 24 is rolled about itself and is positioned within the distal end of the outer shaft 12. In addition, the closure suture 32 and the first and second suture members 34 and 36 are positioned within and extend from a proximal end of the rolled pouch 24 through the channels 22 of the inner shaft 16 to the proximal end of the device 10. In one embodiment, a proximal end of the inner shaft 16 or a distal portion of the gripping member 18 defines a proximal slot or opening 42 (Fig. 3) from which the closure suture 32 and the first and second suture members 34 and 36 extend. The closure suture 32 has a proximal end that is connected to a closure ring 46 and the first and second suture members 34 and 36 have proximal ends that can be tied to first and second knot pullers 48 and 50 (Fig. 8) as will be discussed in further detail below.

Referring to Figs. 5 and 6, when the device 10 is transitioned to the deployed state by advancing the gripping member 18 towards the housing 14 in the direction indicated by arrow "A" in Fig. 5, the inner shaft 16 is advanced within the outer shaft 12 to force the rolled pouch 24 from the outer shaft 12 as indicated by arrow "B" in Fig. 6. Initially, when the pouch 24 exits the outer shaft 12, the pouch 24 is rolled up with the closure member 32 and the first and second suture members 34 and 36 rolled within the pouch 24. The pouch 24 can be unrolled using a grasper (not shown) or other surgical tool to the deployed state shown in Fig. 6. Alternatively, the pouch 24 can be formed of a material which has shape memory characteristics that will automatically move the pouch 24 to an unrolled configuration. Referring to Fig. 7, when the pouch 24 is unrolled to the deployed state, the first suture member 34 extends from the inner shaft 16 and defines a loop 34a dimensioned to be received about body tissue, e.g., an appendix. Similarly, the second suture member 36 extends from the inner shaft 16 and defines a loop 36a which is secured about the open end 28 of the pouch 24 by clips 52 which will be described in further detail below. The loop 34a of the first suture member 34 and/or the loop 36a of the second suture member 36 can be formed from a pre-shaped stiff suture material such as polypropylene or nylon which has a tendency to retain a hoop shape to facilitate placement of tissue through the loop. Alternately, other suture materials can be used to form the first and second suture members.

Referring to Figs. 7 and 8, the proximal end of the closure member 32 is positioned about the open end 28 of the pouch 24. In one embodiment, the pouch 24 defines a series of openings 54 and the closure member 32 is threaded through the openings 54. Although not shown, the distal end of the closure member 32 defines a slip knot (not shown) which facilitates closure of the open end 28 of the pouch 24 as known in the art. When the proximal end of the closure member 32 is pulled proximally by pulling proximally on the closure ring 46, the knot (not shown) in the closure member 32 engages the distal end of a respective internal channel 22 (Fig. 2) of the inner tube 16 to close the open end of the pouch 24 as is known in the art.

As shown in Fig. 8, the knot pullers 48 and 50 are slidably supported about the proximal end of the inner shaft 16. The proximal end of the first suture member 34 is secured to the knot puller 48 and the proximal end of the second suture member 36 is secured to the knot puller 50. Each of the first and second knot pullers 48 and 50 can be manually retracted from an advanced position to a retracted position to withdraw the respective suture member 34, 36 into a respective channel 22 (Fig. 2) of the inner shaft 16. As discussed above with regard to the closure member 32, each of the distal ends of the suture members 34 and 36 defines a knot (not shown) which engages a distal end of a respective channel 22 of the inner shaft 16 as the suture member 34, 36 is retracted into the inner shaft 16 to cinch the loop 32a, 34a as the first and second knot pullers 48 and 50 are moved to their retracted positions.

In embodiments, each of the knot pullers 48 and 50 has a semi-circular body 60 and a proximally extending finger 62. The proximal end of the first suture member 32 is tied to the finger 62 of the first knot puller 48 and the proximal end of the second suture member 34 is tied to the finger of the second knot puller 50. The semi-circular bodies 60 of the first and second knot pullers 48 and 50 are slidably positioned about the proximal end of the inner shaft 16 to facilitate independent movement of each of the knot pullers 48 and 50 about the inner shaft 16. It is noted that the fingers 62 may be formed from a deformable material which deforms outwardly when the tension in a suture member exceeds a predetermined force to prevent the respective suture loop 34a, 36a from cutting through tissue.

Referring briefly to Fig. 7, when the pouch 24 is moved to the deployed state, the first suture member 34 extends from the distal end of the inner shaft 16 and is unattached to the pouch 24 and movable in relation to the pouch 24. In contrast, the loop 36a of the second suture member 36 is supported about the open end 28 of the pouch 24 by the clips 52. Clips 52 can be integrally formed with the pouch 24 and define a hook or U-shape configured to releasably engage the loop 36a (Fig. 10A). Other securement members are envisioned for releasably securing the second suture member 36 to the pouch 24.

Referring to Figs. 9-10A, in use, the outer shaft 12 of the retrieval device 10 is inserted through a cannula (not shown) to a position adjacent a surgical site in its non-deployed state (Fig. 1). It is noted that during endoscopic surgical procedures, the area within the patient where the surgical procedure is to take place, e.g., the abdomen, may be insufflated to provide space for the procedure to be conducted. Once at the surgical site, the retrieval device 10 is moved to the deployed state by moving the gripping member 18 towards the housing 14 (Fig. 5) to advance the inner shaft 16 within the outer shaft 12. As the inner shaft 16 moves within the outer shaft 12, the distal end of the inner shaft 16 engages the proximal end of the rolled pouch 24 and pushes the rolled pouch 24 from the distal end of the outer shaft 12. Next, the pouch 24 can be unrolled using for, e.g., a surgical grasper, to expose the open end 28 of the pouch 24 (Fig. 7). Alternatively, as discussed above, the pouch can be formed of a material or be embedded with a resilient material which allows the pouch 24 to automatically unroll upon exiting the outer shaft 12.

After the pouch has been unrolled at the surgical site, a surgical grasper or the like can be used to manipulate tissue "T", e.g., an appendix, to position the tissue "T" through the loop 34a of the first suture member 34. The tissue "T" is passed through the open end 28 of the pouch 24 into the receptacle 26. After tissue is passed through the loop 34a of the first suture 34, the first knot puller 48 can be retracted in the direction indicated by arrow "C" in Fig. 9 about the proximal end of the inner shaft 16 or gripping member 18 to cinch and knot the first suture loop 34a about the tissue "T" (Fig. 10). The first suture member 34 can now be used to manipulate the tissue "T" to assist the clinician in positioning the tissue within the pouch 24 (Fig. 10). In this position, the second suture member 36 is retained to the pouch 24 by clips 52 (Fig. 10A).

Referring to Figs. 11-12, after the tissue "T" is positioned within the receptacle 26 of the pouch 24 and the loop 34a is cinched, the second knot puller 50 can be retracted about the inner shaft 16 in the direction indicated by arrow "B" to cinch and knot the loop 36a of the second suture member 36 about the tissue "T". As the loop 36a is cinched, the suture 36 is pulled from the clips 52 to separate the suture loop 36a from clips 52 of the pouch 24 (Fig. 11A). Thereafter, the tissue "T" can be transected between the two suture members 34 and 36 to allow the tissue "T" to fall into the pouch 24.

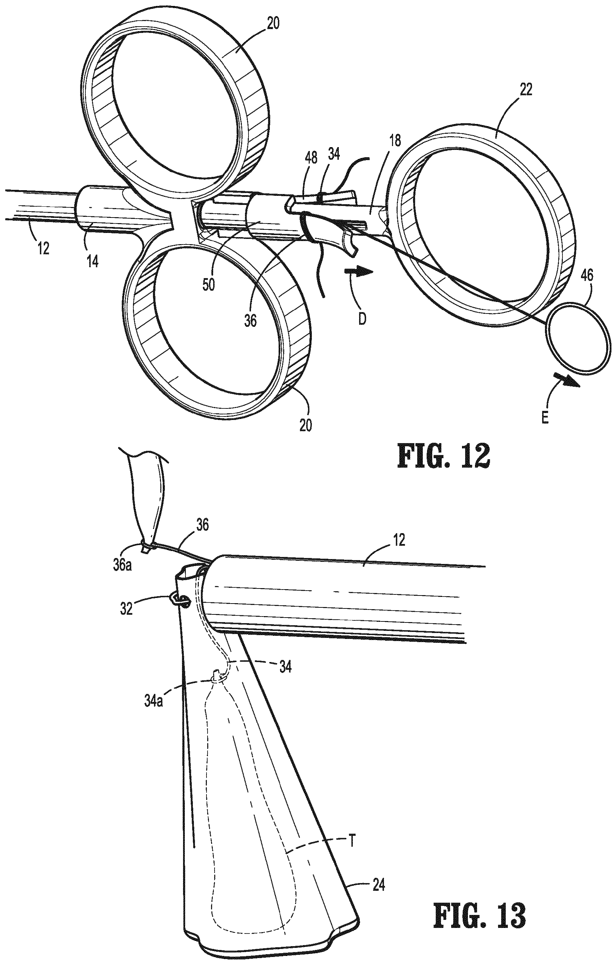

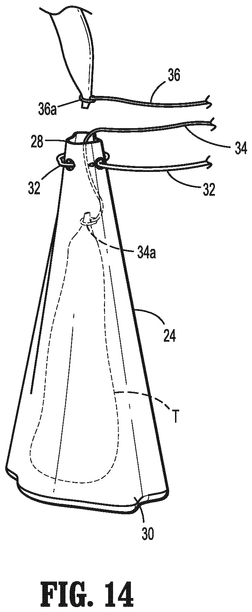

Referring to Figs. 12-14, after the tissue "T" is placed within the pouch 24, the closure ring 46 is pulled proximally in the direction indicated by arrow "E" in Fig. 12 to cinch the open end 28 of the pouch 24 and enclose the tissue "T" within the pouch 24. Thereafter, the proximal end of the suture members 34 and 36 can be separated from the knot pullers 48 and 50 and the closure ring 46 can be separated from the closure member 32 to facilitate separation of the inner and outer shafts 12, 16 of the retrieval device 10 from the pouch 24 as shown in Fig 14.

The presently disclosed ligation and specimen retrieval device 10 provides a number of advantages over known endoscopic instruments. For example, the device 10 reduces the risk of infection by securing the tissue within the pouch 24 before the tissue is dissected from healthy tissue. In addition, the device 10 reduces the time required to perform a surgical procedure by reducing the number of instrument exchanges, reducing the number of steps required to perform the procedure and by reducing the amount of tissue manipulation required to complete the procedure. The cost of the procedure is also reduced because the number of instruments required to complete the procedure is reduced as well is the time required to complete the procedure. Further, because of its simplicity, the device 10 can be dimensioned to be received within a 5 mm cannula.

While several embodiments of the disclosure have been shown in the drawings, it is not intended that the disclosure be limited thereto, as it is intended that the disclosure be as broad in scope as the art will allow and that the specification be read likewise. Any combination of the above embodiments is also envisioned and is within the scope of the appended claims. Therefore, the above description should not be construed as limiting, but merely as exemplifications of particular embodiments. Those skilled in the art will envision other modifications within the scope of the claims appended hereto.

## Claims

1. A ligation and specimen retrieval device (10) comprising:
a hollow outer shaft (12) having a proximal end and a distal end;
an inner shaft (16) extending through the outer shaft, the inner shaft being movable in relation to the outer shaft from a retracted position to and advanced position;
a pouch (24) defining a receptacle (26), the pouch having an open end (28) and a closed end (30);
a pouch closure member (32) extending through the outer shaft, the pouch closure member having a proximal end extending from a proximal end of the outer shaft and a distal end positioned about the open end of the pouch, wherein the pouch closure member is retractable within the outer shaft to cinch the open end of the pouch; **characterised by** at least one pair of suture members (34, 36) extending from the proximal end of the outer shaft to the distal end of the outer shaft, each suture member including a distal end having a suture loop and a proximal end that is accessible to a clinician at the proximal end of the outer shaft, wherein each suture member is adapted to be cinched about tissue upon retraction of the suture member within the outer tube;
the ligation and specimen retrieval device being transitionable from a non-deployed state in which the pouch and the suture members are supported within a distal end of the outer shaft to a deployed state in which the pouch and the suture members are positioned externally of the outer shaft.

2. The ligation and specimen retrieval device of claim 1, wherein in the non-deployed state, the pouch (24) is rolled up and supported in the distal end of the outer shaft (12).

3. The ligation and specimen retrieval device of claim 2, wherein in the non-deployed state, the pouch (24) is rolled about at least one suture member (34).

4. The ligation and specimen retrieval device of any one of the preceding claims, wherein the second suture loop (36) is secured to the pouch (24) adjacent the open end (28) of the pouch.

5. The ligation and specimen retrieval device of claim 4, wherein the pouch (24) includes a plurality of clips (52) which releasably secure the second suture loop (36) to the pouch.

6. The ligation and specimen retrieval device of claim 5, wherein the clips (52) are configured to automatically release the second suture loop (36) upon retraction of the second suture member within the outer shaft.

7. The ligation and specimen retrieval device of any one of the preceding claims, wherein the proximal end of the first and second suture members (34, 36) are secured to first and second knot pullers (48, 50), the first and second knot pullers being movably supported on the proximal end of the ligation and specimen retrieval device from an advanced position to a retracted position to retract the first and second suture members within the outer tube.

8. The ligation and specimen retrieval device of any preceding claim, wherein the inner shaft (16) defines at least one channel (22) and the suture members extend through the at least one channel from the distal end of the inner shaft to the proximal end of the inner shaft.

9. The ligation and specimen retrieval device of any preceding claim, further including a gripping member (18) secured to a proximal end of the inner shaft (16).

10. The ligation and specimen retrieval device of claim 9, further including a housing (14) secured to a proximal end of the outer shaft, the housing having a pair of finger loops (20) and the gripping member having a thumb loop (22), the finger loops and the thumb loop being configured to facilitate movement of the inner shaft (16) in relation to the outer shaft (12) from the retracted position to the advanced position.

11. The ligation and specimen retrieval device of any one of the preceding claims, wherein the proximal end of the pouch closure member (32) includes a closure ring (46) that is configured to be grasped by a clinician.

## Patentansprüche

1. Ligatur- und Probenbeschaffungsvorrichtung (10), umfassend:
einen hohlen äußeren Stiel (12), der ein proximales Ende und ein distales Ende aufweist;
einen inneren Stiel (16), der sich durch den äußeren Stiel erstreckt, wobei der innere Stiel in Bezug auf den äußeren Stiel von einer zurückgezogenen Position in eine vorgerückte Position bewegbar ist;
eine Tasche (24), die einen Behälter (26) definiert, wobei die Tasche ein offenes Ende (28) und ein geschlossenes Ende (30) aufweist;
ein Taschenverschlusselement (32), das sich durch den äußeren Stiel erstreckt, wobei das Taschenverschlusselement ein proximales Ende, das sich von einem proximalen Ende des äußeren Stiels erstreckt, und ein distales Ende, das um das offene Ende der Tasche positioniert ist, aufweist, wobei das Taschenverschlusselement innerhalb des äußeren Stiels zurückziehbar ist, um das offene Ende der Tasche zu sichern; **gekennzeichnet durch** mindestens ein Paar von Fadenelementen (34, 36), die sich von dem proximalen Ende des äußeren Stiels zu dem distalen Ende des äußeren Stiels erstrecken, wobei jedes Fadenelement ein distales Ende einschließt, das eine Fadenschleife, und ein proximales Ende, das von einem Mediziner an dem proximalen Ende des äußeren Stiels zugänglich ist, aufweist, wobei jedes Fadenelement geeignet ist, um Gewebe herum beim Zurückziehen des Fadenelements innerhalb der äußeren Röhre gesichert zu werden;
wobei die Ligatur- und Probenbeschaffungsvorrichtung von einem nicht-eingesetzten Zustand, in dem die Tasche und die Fadenelemente innerhalb eines distalen Endes des äußeren Stiels gestützt sind, in einen eingesetzten Zustand, in dem die Tasche und die Fadenelemente außerhalb des äußeren Stiels positioniert sind, überführbar ist.

2. Ligatur- und Probenbeschaffungsvorrichtung nach Anspruch 1, wobei in dem nicht-eingesetzten Zustand, die Tasche (24) aufgerollt und in dem distalen Ende des äußeren Stiels (12) gestützt ist.

3. Ligatur- und Probenbeschaffungsvorrichtung nach Anspruch 2, wobei in dem nicht-eingesetzten Zustand die Tasche (24) mindestens um ein Fadenelement (34) herum aufgerollt ist.

4. Ligatur- und Probenbeschaffungsvorrichtung nach einem der vorstehenden Ansprüche, wobei die zweite Fadenschleife (36) an der Tasche (24) benachbart zu dem offenen Ende (28) der Tasche gesichert ist.

5. Ligatur- und Probenbeschaffungsvorrichtung nach Anspruch 4, wobei die Tasche (24) eine Vielzahl von Klammern (52) einschließt, die lösbar die zweite Fadenschleife (36) an der Tasche sichern.

6. Ligatur- und Probenbeschaffungsvorrichtung nach Anspruch 5, wobei die Klammern (52) eingerichtet sind, um automatisch die zweite Fadenschleife (36) beim Rückziehen des zweiten Fadenelements innerhalb des äußeren Stiels zu lösen.

7. Ligatur- und Probenbeschaffungsvorrichtung nach einem der vorstehenden Ansprüche, wobei das proximale Ende der ersten und zweiten Fadenelemente (34, 36) an ersten und zweiten Knotenziehern (48, 50) gesichert sind, wobei die ersten und zweiten Knotenzieher bewegbar auf dem proximalen Ende der Ligatur- und Probenbeschaffungsvorrichtung von einer vorgerückten Position zu einer zurückgezogenen gestützt sind, um die ersten und zweiten Fadenelemente innerhalb der äußeren Röhre zurückzuziehen.

8. Ligatur- und Probenbeschaffungsvorrichtung nach einem der vorstehenden Ansprüche, wobei der innere Stiel (16) mindestens einen Kanal (22) definiert und die Fadenelemente sich durch den mindestens einen Kanal von dem distalen Ende des inneren Stiels zu dem proximalen Ende des inneren Stiels erstecken.

9. Ligatur- und Probenbeschaffungsvorrichtung nach einem der vorstehenden Ansprüche, weiter einschließend ein Greifelement (18), das an einem proximalen Ende des inneren Stiels (16) gesichert ist.

10. Ligatur- und Probenbeschaffungsvorrichtung nach Anspruch 9, weiter einschließend ein Gehäuse (14), das an einem proximalen Ende des äußeren Stiels gesichert ist, wobei das Gehäuse ein Paar von Fingerschleifen (20) aufweist und das Greifelement eine Daumenschleife (22) aufweist, wobei die Fingerschleifen und die Daumenschleife eingerichtet sind, um eine Bewegung des inneren Stiels (16) in Bezug auf den äußeren Stiel (12) von der zurückgezogenen Position zu der vorgerückten Position zu erleichtern.

11. Ligatur- und Probenbeschaffungsvorrichtung nach einem der vorstehenden Ansprüche, wobei das proximale Ende des Taschenverschlusselements (32) einen Verschlussring (46) einschließt, der eingerichtet ist, um von einem Mediziner gegriffen zu werden.

## Revendications

1. Dispositif d'extraction d'échantillons et de ligature (10) comprenant :
une tige externe creuse (12) ayant une extrémité proximale et une extrémité distale ;
une tige interne (16) s'étendant à travers la tige externe, la tige interne étant mobile par rapport à la tige externe d'une position rétractée à une position avancée ;
une poche (24) définissant un réceptacle (26), la poche ayant une extrémité ouverte (28) et une extrémité fermée (30) ;
un élément de fermeture de poche (32) s'étendant à travers la tige externe, l'élément de fermeture de poche ayant une extrémité proximale s'étendant d'une extrémité proximale de la tige externe et une extrémité distale positionnée autour de l'extrémité ouverte de la poche, dans lequel l'élément de fermeture de poche est rétractable dans la tige externe pour serrer l'extrémité ouverte de la poche ; **caractérisé par** au moins une paire d'éléments de suture (34, 36) s'étendant de l'extrémité proximale de la tige externe à l'extrémité distale de la tige externe, chaque élément de suture comprenant une extrémité distale ayant une boucle de suture et une extrémité proximale qui est accessible à un médecin à l'extrémité proximale de la tige externe, dans lequel chaque élément de suture est à même d'être serré autour d'un tissu lors de la rétraction de l'élément de suture dans le tube externe ;
le dispositif d'extraction d'échantillons et de ligature pouvant effectuer une transition entre un état non déployé, dans lequel la poche et les éléments de suture sont supportés dans une extrémité distale de la tige externe, à un état déployé, dans lequel la poche et les éléments de suture sont positionnés à l'extérieur de la tige externe.

2. Dispositif d'extraction d'échantillons et de ligature selon la revendication 1, dans lequel, à l'état non déployé, la poche (24) est enroulée et supportée à l'extrémité distale de la tige externe (12).

3. Dispositif d'extraction d'échantillons et de ligature selon la revendication 2, dans lequel, à l'état non déployé, la poche (24) est enroulée autour d'au moins un élément de suture (34).

4. Dispositif d'extraction d'échantillons et de ligature selon l'une quelconque des revendications précédentes, dans lequel la seconde boucle de suture (36) est fixée à la poche (24) de manière adjacente à l'extrémité ouverte (28) de la poche.

5. Dispositif d'extraction d'échantillons et de ligature selon la revendication 4, dans lequel la poche (24) comprend une pluralité d'agrafes (52) qui fixent de manière libérable la seconde boucle de suture (36) à la poche.

6. Dispositif d'extraction d'échantillons et de ligature selon la revendication 5, dans lequel les agrafes (52) sont configurées pour libérer automatiquement la seconde boucle de suture (36) lors de la rétraction du second élément de suture dans la tige externe.

7. Dispositif d'extraction d'échantillons et de ligature selon l'une quelconque des revendications précédentes, dans lequel l'extrémité proximale du premier et du second élément de suture (34, 36) est fixée à un premier et un second noueur (48, 50), les premier et second tireurs noueurs pouvant être supportés de manière mobile sur l'extrémité proximale du dispositif d'extraction d'échantillons et de ligature d'une position avancée à une position rétractée pour rétracter les premier et second éléments de suture dans le tube externe.

8. Dispositif d'extraction d'échantillons et de ligature selon l'une quelconque des revendications précédentes, dans lequel la tige interne (16) définit au moins un canal (22) et les éléments de suture s'étendent à travers au moins un canal de l'extrémité distale de la tige interne à l'extrémité proximale de la tige interne.

9. Dispositif d'extraction d'échantillons et de ligature selon l'une quelconque des revendications précédentes, comprenant en outre un élément de préhension (18) fixé à une extrémité proximale de la tige interne (16).

10. Dispositif d'extraction d'échantillons et de ligature selon la revendication 9, comprenant en outre un boîtier (14) fixé à une extrémité proximale de la tige externe, le boîtier ayant une paire de boucles de doigts (20) et l'élément de préhension ayant une boucle de pouce (22), les boucles de doigts et la boucle de pouce étant configurées pour faciliter le mouvement de la tige interne (16) par rapport à la tige externe (12) de la position rétractée à la position avancée.

11. Dispositif d'extraction d'échantillons et de ligature selon l'une quelconque des revendications précédentes, dans lequel l'extrémité proximale de l'élément de fermeture de poche (32) comprend un anneau de fermeture (46) qui est configuré pour être saisi par un médecin.
